(19) Europäisches Patentamt
European Patent Office
Office européen des brevets

(11) Veröffentlichungsnummer: **0 168 607**
**B1**

(12) # EUROPÄISCHE PATENTSCHRIFT

(45) Veröffentlichungstag der Patentschrift: **08.02.89**

(51) Int. Cl.⁴: **C 07 D 207/16**

(21) Anmeldenummer: **85106797.5**

(22) Anmeldetag: **03.06.85**

(54) **Neue Pyrrolidincarbonsäurederivate, Verfahren zu ihrer Herstellung.**

(30) Priorität: **08.06.84 DE 3421295**

(43) Veröffentlichungstag der Anmeldung: **22.01.86 Patentblatt 86/4**

(45) Bekanntmachung des Hinweises auf die Patenterteilung: **08.02.89 Patentblatt 89/6**

(84) Benannte Vertragsstaaten: **AT BE CH DE FR GB IT LI NL SE**

(56) Entgegenhaltungen:
**FR-A- 1 461 413**
**US-A- 3 274 212**

**THE JOURNAL OF ORGANIC CHEMISTRY, Band 41, Nr. 2, 23. Januar 1976, Seiten 192-195; R.A. KRETCHMER u.a.: "Formation of 2-oxazolines by a cyclization involving the displacement of mercury"**
**THE JOURNAL OF ORGANIC CHEMISTRY, Band 41, Nr. 21, 15. Oktober 1976, Seiten 3491-3493; G. STORK u.a.: "Alkylation and Michael additions of glycine ethyl ester. Use in alpha-amino acid synthesis and as acyl carbanion equivalent"**
**TETRAHEDRON LETTERS, Nr. 30, Juli 1978, Seiten 2641-2644, Pergamon Press Ltd., Oxford, GB; M.J. O'DONNELL u.a.: "The synthesis of amino acids by phase-transfer reactions"**

(73) Patentinhaber: **BASF Aktiengesellschaft, Carl-Bosch-Strasse 38, D-6700 Ludwigshafen (DE)**

(72) Erfinder: **Schulz, Guenter, Dr., Carl-Bosch-Strasse 96, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 168 607 B1

ACTORUM AG

## Beschreibung

Es ist bekannt, dass Schiff-Basen von Glycinestern unter Einwirkung von Basen und Alkylierungsmitteln an der $CH_2$-Gruppe alkyliert werden können (z.B. M.J. O'Donnell, J.M. Boniece and S.E. Earp, Tetr. Letters 1978 (30), 2641–2644). Es ist weiterhin bekannt, dass in protischen Solventien die Michael-Addition von Anionen der Glycinester-Schiff-Basen an α,β-ungesättigte Ketone oder an Acrylester-Derivate gelingt (G. Stork, A.Y.W. Leong, A.M. Touzin, J. Org. Chem. 41, 3491 (1976) und Zitate hier). In allen bekannten Fällen konnten immer nur offenkettige Verbindungen erhalten werden.

Nun besteht eine ganz allgemeine Aufgabe der Chemie darin, heterocyclische und insbesondere weitgehend substituierte heterocyclische Verbindungen zu schaffen, weil solche Verbindungen wichtige Zwischenprodukte für die Herstellung z.B. von Pharmazeutika oder Wirkstoffen auf dem Gebiet des Pflanzenschutzes darstellen. Die Bereitstellung neuer heterocyclischer Verbindungen ermöglicht auch die Herstellung neuer Folgeprodukte; z.T. sind die heterocyclischen Verbindungen selbst schon biologisch aktiv. Die Erfindung löst eine solche Aufgabe, indem sie gegebenenfalls substituierte Pyrrolidincarbonsäure(ester) und Verfahren zu ihrer Herstellung schafft.

Es wurde ein Verfahren gefunden, das, allgemein gesprochen, die Umsetzung von Schiff-Basen von Glycinestern mit Elektronacceptor-substituierten Doppelbindungen zu cyclischen Verbindungen ermöglicht.

Gegenstand der Erfindung sind Pyrrolidin-2-carbonsäure-Derivate der Formel I

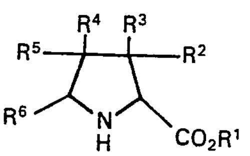

I,

in welcher

$R^1$, $R^2$, $R^3$ unabhängig voneinander für Wasserstoff, nieder-Alkyl mit bis zu 5 Kohlenstoffatomen, nieder-Halogenalkyl mit bis zu 3 Kohlenstoffatomen und bis zu 5 Halogenatomen, Phenylalkyl mit bis zu 4 Kohlenstoffatomen im Alkylteil, Halogenphenylalkyl mit bis zu 3 Halogenatomen im Phenylteil und bis zu 4 Kohlenstoffatomen im Alkylteil stehen, $R^2$ und $R^3$ darüber hinaus unabhängig voneinander für substituiertes Phenyl stehen, wobei als Substituenten bis zu 3 Halogenatome, eine Nitro-, eine Cyano- oder eine Alkoxygruppe mit bis zu 4 Kohlenstoffatomen gemeint sind, und darüber hinaus $R^3$ auch für unsubstituiertes Phenyl stehen kann,

$R^4$ für Wasserstoff, n- oder i-Alkyl mit bis zu 5 Kohlenstoffatomen, Phenylalkyl mit bis zu 4 Kohlenstoffatomen im Alkylteil, Halogenphenylalkyl mit bis zu 4 Halogenatomen im Phenylteil und bis zu 4 Kohlen stoffatomen im Alkylteil, Phenyl oder substituiertes Phenyl, wobei als Substituenten bis zu 3 Halogenatome, eine Nitro-, eine Cyano- oder eine Alkoxygruppe mit bis zu 4 Kohlenstoffatomen gemeint sind, einen fünf- bis sechsatomigen Heterocyclus mit bis zu 3 Stickstoffen als Heteroatomen, eine Cyanogruppe, eine Alkylcarbonylgruppe mit bis zu 5 Kohlenstoffatomen, eine Halogenalkylcarbonylgruppe mit bis zu 3 Kohlenstoffatomen und bis zu 5 Halogenatomen oder eine Alkoxycarbonylgruppe mit bis zu 5 Kohlenstoffatomen steht,

$R^5$ für Alkylcarbonyl mit bis zu 6 Kohlenstoffatomen, Halogenalkylcarbonyl mit bis zu 3 Kohlenstoffatomen und bis zu 5 Halogenatomen, Alkoxycarbonyl mit bis zu 5 Kohlenstoffatomen, gegebenenfalls substituiertes Phenylcarbonyl, wobei als Substituenten bis zu 3 Halogenatome, eine Alkoxygruppe mit bis zu 4 Kohlenstoffatomen, eine Alkylthiogruppe mit bis zu 4 Kohlenstoffatomen, eine Cyano- oder eine Nitrogruppe gemeint sind, eine Cyano- oder eine Nitrogruppe steht,

$R^6$ für gegebenenfalls substituiertes Phenyl, wobei als Substituenten bis zu 3 Halogenatome, eine Alkoxygruppe mit bis zu 4 Kohlenstoffatomen, eine Alkylthiogruppe mit bis zu 4 Kohlenstoffatomen, eine Alkylgruppe mit bis zu 5 Kohlenstoffatomen, eine Cyano- oder eine Nitrogruppe gemeint sind, oder tert. Alkyl mit bis zu 5 Kohlenstoffatomen steht.

Gegenstand der Erfindung sind weiterhin Pyrrolidincarbonsäurederivate Ia

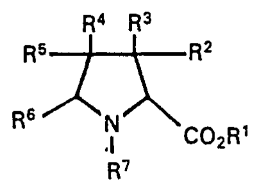

Ia,

wobei $R^7$ für nieder-Alkyl mit bis zu 5 Kohlenstoffatomen, nieder-Alkenyl mit bis zu 5 Kohlenstoffatomen, das mit bis zu 3 Halogenatomen substituiert ist, nieder-Alkinyl mit bis zu 5 Kohlenstoffatomen, gegebenenfalls substituiertes Phenylalkyl mit bis zu 3 C-Atomen im Alkylteil und bis zu 3 Halogenatomen, einer Cyano- oder einer Nitrogruppe als Substituenten im Phenylteil, ggf. substituiertes Alkylcarbonyl mit bis zu 5 Kohlenstoffatomen und bis zu 3 Halogenatomen oder einer Alkoxygruppe ($C_1$–$C_3$) als Substituenten, Alkoxycarbonyl mit bis zu 5 Kohlenstoffatomen, gegebenenfalls substituiertes Phenylcarbonyl mit bis zu 3 Halogenatomen als Substituenten,

Phenylalkylcarbonyl mit bis zu 3 Kohlenstoffatomen im Alkylteil oder Benzyloxycarbonyl steht, und $R_1$–$R_6$ die bei der Definition für Formel I angegebenen Bedeutungen haben.

Als Beispiele für solche Pyrrolidin-2-carbonsäurederivate der Formel Ia seien besonders solche Verbindungen genannt, in denen

$R^1$, $R^2$ und $R^3$ unabhängig voneinander für Wasserstoff, Methyl, Ethyl, Propyl, tert.-Butyl, Trifluormethyl, Benzyl, 4-Chlorbenzyl stehen und

$R^2$ und $R^3$ darüber hinaus unabhängig voneinander für 2,4-Dichlorphenyl, 4-Methoxyphenyl und 4-Nitrophenyl stehen,

$R^4$ für Wasserstoff, Methyl, Cyano, Phenyl, Benzyl, 4-Chlorbenzyl, 1-(1,2,4-Triazolyl), 1-Imidazolyl, Acetyl, Methoxycarbonyl und Ethoxycarbonyl steht,

$R^5$ für Acetyl, Ethoxycarbonyl, 2,4-Dichlorbenzoyl, 4-Chlorbenzoyl, 4-Brombenzoyl, 4-Methoxybenzoyl, 4-Propoxybenzoyl, 4-Butoxybenzoyl, 4-Cyanobenzoyl, 4-Methylthiobenzoyl oder 4-Nitrobenzoyl steht,

$R^6$ für Phenyl, 4-Chlorphenyl, 4-Nitrophenyl, 4-Methylthiophenyl oder 4-Cyanophenyl steht,

$R^7$ für Methyl, Ethyl, Allyl, 2,3-Dichlorallyl, 2,3,3-Trichlorallyl, Propinyl, Benzyl, 4-Chlorbenzyl, 2,4-Dichlorbenzyl, 4-Fluorbenzyl, 4-Cyanobenzyl, Acetyl, Methoxyacetyl, Chloracetyl, Methoxycarbon, Ethoxycarbonyl, Benzyloxycarbonyl, Benzoyl, 4-Chlorbenzoyl, 3,5-Dichlorbenzoyl oder 2,4-Dichlorbenzoyl steht.

Die Verbindungen der Formel Ia werden durch Acylierung oder Alkylierung der Pyrrolidine der Formel I erhalten.

Die Erfindung betrifft somit die Addition von Schiff-Basen von Glycinestern des Typs (V) an durch elektronenziehende Substituenten aktivierte Doppelbindungen des Typs (IV), die nach dem folgenden praktischen Beispiel cyclische Verbindungen der Formel I liefert.

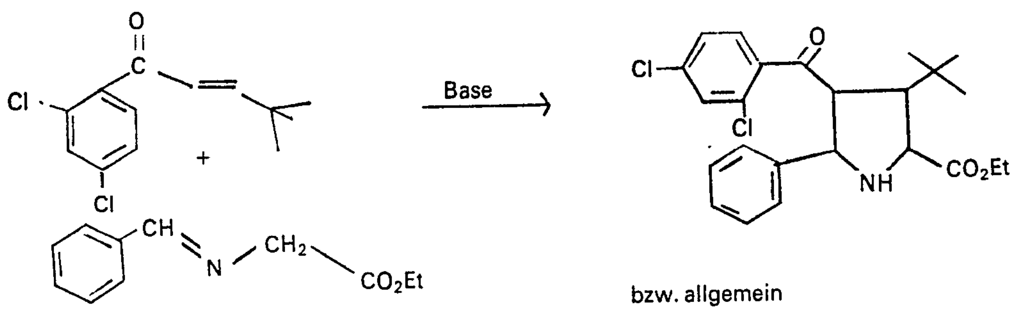


bzw. allgemein

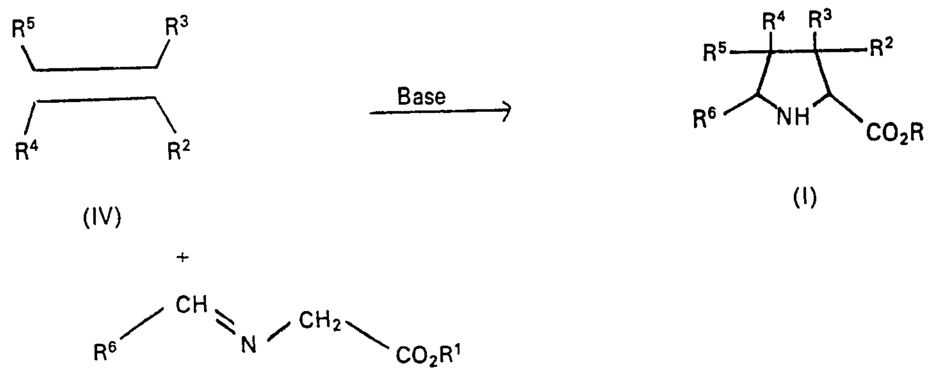


(IV)

(I)

(V)

Die Bildung cyclischer Verbindungen ist überraschend, da, wie gesagt, bisher für ähnliche Umsetzungen die offenkettigen einfachen Michael-Addukte beschrieben wurden. Diese neue Synthese von 2-Pyrrolidincarbonsäurederivaten ist ökonomisch besonders günstig, weil in ihrem Verlauf zwei Bindungen in einem Zug geknüpft wurden, so dass man ein Cycloadditionsprodukt erhält. Ausserdem zeichnet sich das neue Verfahren durch besonders einfache Durchführbarkeit und leicht einzuhaltende Randbedingungen für die Reaktion aus.

Das neue Verfahren zur Synthese von Derivaten der 2-Pyrrolidincarbonsäure ist dadurch gekennzeichnet, dass – vorzugsweise stöchiometrisch – Mengen eines Alkens der Formel IV und einer Schiff-Base der Formel V durch Zusatz einer Base nach dem vorstehenden Schema zur Reaktion gebracht werden. Als Basen sind dabei einfache Alkoholate oder tertiäre Amine gut geeignet; z.B. kann man Natriummethylat oder Natriumethylat, Kaliumtertiärbutylat oder Triethylamin einsetzen. Die Base soll in einer geringeren als der stöchiometrischen Menge, z.B. 10 bis 70% der äquimolaren Menge zugegen sein. 10 bis 30% der äquimolaren Menge seien als Beispiel besonders genannt. Die Umsetzung kann in unverdünntem Reaktionsgemisch oder unter Verwendung eines Verdünnungsmittels durchgeführt werden. Als Verdünnungsmittel sind protische organische Lösungsmittel wie z.B. Methanol, Ethanol, i-Propanol oder tert.-Butanol gut geeignet. Die Verwendung von Verdünnungsmitteln kann für die Ausführung der Umsetzung und für die Aufarbeitung Vorteile bringen, wenn die Reaktanden löslich sind und das sich bildende 2-Pyrrolidincarbonsäurederivat ausfällt. Die Umsetzung kann bei Raumtemperatur oder bei erhöhter Temperatur durchgeführt werden. Als Beispiel sei die Umsetzung in Ethanol genannt, bei der Temperaturen zwischen 60 °C und Siedetemperatur des Lösungsmittels günstig für kurze Reaktionszeiten und gute Ausbeuten sind.

Die Pyrrolidincarbonsäurederivate der Formel I können durch Alkylierung oder Acylierung in die Pyrrolidincarbonsäurederivate der Formel Ia übergeführt werden:

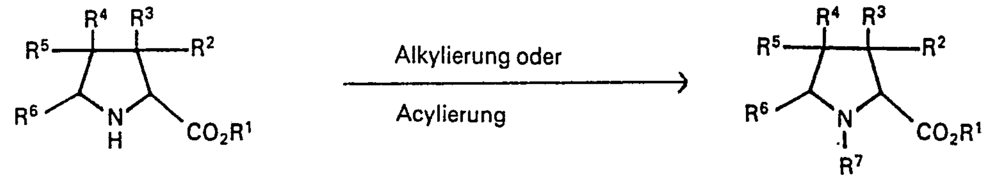


(I) (Ia,)

Die Decarboxylierung von Verbindungen I bzw. Ia ist möglich und führt zu entsprechenden Pyrrolidinen.

Geeignete Alkylierungs- bzw. Acylierungsmittel zur Gewinnung der Verbindungen des Typs Ia sind z.B. Verbindungen des Typs $R^7X$, wobei X Cl, Br, I, Tosylat, Mesylat oder Sulfat bedeutet. Als Beispiel für Alkylierung mittel $R^7X$ seien folgende Verbindungen genannt: Methyljodid, Allylchlorid, 2,3-Dichlorallylchlorid, 2,3,3-Trichlorallylchlorid, Propinylchlorid, Benzylchlorid, 4-Chlorbenzylchlorid, 2,4-Dichlorbenzylchlorid, 4-Fluorbenzylchlorid, 4-Cyanobenzylchlorid, Dimethylsulfat, Diethylsulfat.

Bei der Acylierung bedeutet X eine aktivierte Carbonsäuregruppe CO–Y, in welcher Y z.B. für Cl, Br, 1-Imidazolyl $CF_3CO_2$– oder $C_1$– bis $C_4$-Monoalkylcarbonat steht. Als Beispiele für Acylierungsmittel $R^7COY$ seien folgende Verbindungen genannt: Acetylchlorid, Methoxyacetylchlorid, Trifluoressigsäureanhydrid, Chloracetylchlorid, Chlorkohlensäuremethylester, Chlorkohlensäureethylester, Benzoylchlorid, 4-Chlorbenzoylchlorid, 3,5-Dichlorbenzoylchlorid, 2,4-Dichlorbenzoylchlorid.

Die Umsetzung erfolgt bei Raumtemperatur oder bei erhöhter Temperatur in Gegenwart eines organischen Säurefängers und im Falle der Acylierung ggf. in Gegenwart eines Acylierungskatalysators oder in Gegenwart eines anorganischen Säurefängers. Als organische Säurefänger sind tertiäre Amine oder aromatische Stickstoff-Basen geeignet, beispielsweise Triethylamin bzw. Pyridin. 4-Dimethylaminopyridin kann gegebenenfalls als Acylierungskatalysator eingesetzt werden. Als anorganische Säurefänger sind Alkali- oder Erdalkalicarbonate geeignet, z.B. Kaliumcarbonat bzw. Calciumcarbonat. Als Verdünnungsmittel sind niedere Alkohole oder aprotisch dipolare organische Solventien geeignet, z.B. t-Butanol bzw. DMSO, DMF und N-Methylpyrrolidon.

Verbindungen der Formel Ib

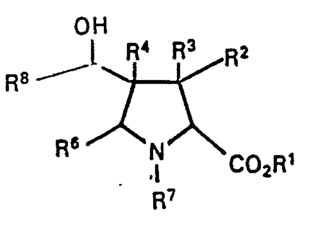

Ib,

können aus geeigneten Pyrrolidin-Derivaten I oder Ia durch Reduktion mit komplexen Metallhydriden hergestellt werden.

Für die Umsetzung geeignete Verbindungen sind alle unter I bzw. Ia genannten Verbindungen mit der Massgabe, dass $R^4$ nur für n- oder i-Alkyl, ggf. subst. Phenylalkyl, ggf. subst. Phenyl, einen fünf- bis sechsgliedrigen aromatischen Heterocyclus mit bis zu 3 Stickstoffatomen oder eine Cyanogruppe steht, und mit der Massgabe, dass $R^5$ nur für ggf. subst. Alkylcarbonyl oder ggf. subst. Phenylcarbonyl steht.

In Formel Ib haben $R^1$, $R^2$, $R^3$, $R^4$, $R^6$, $R^7$ die bei der Definition für Formel I bzw. Ia angegebenen Bedeutungen, mit der Massgabe, dass $R^4$ die oben genannten Bedeutungen hat und mit der Massgabe, dass $R^7$ auch H sein kann.

$R^8$ bedeutet in Formel Ib Alkyl oder Halogenalkyl mit bis zu 4 Kohlenstoffatomen und bis zu 3 Halogenatomen oder gegebenenfalls substituiertes Phenyl, wobei «Substituenten» bis zu 3 Halogenatome, eine Alkoxygruppe mit bis zu 4 Kohlenstoffatomen, eine Alkylthiogruppe mit bis zu 4 Kohlenstoffatomen, eine Cyano- oder eine Nitrogruppe bedeutet. Als Beispiele für $R^8$ seien Phenyl, 4-Chlorphenyl, 2,4-Dichlorphenyl, 4-Methoxyphenyl, 4-Ethoxyphenyl, 4-Propoxyphenyl, 4-Butoxyphenyl, 4-Cyanophenyl, 4-Nitrophenyl genannt.

Als komplexes Hydrid zur Reduktion ist vorzugsweise Natriumborhydrid geeignet. Die Umsetzung erfolgt in protischen organischen Lösungsmitteln wie Methanol, Ethanol oder i-Propanol, vorzugsweise in Methanol.

Aus der nachstehenden Tabelle typischer Verbindungen sind einige repräsentative Herstellungsbeispiele für die neuen Verbindungen herausgegriffen.

Die übrigen Verbindungen der Tabelle sind, soweit sie durch physikalische Angaben (Schmelzpunkt, Aggregatzustand) charakterisiert sind, durch sinngemässe Abwandlung der Beispiele erhalten worden.

Herstellungsbeispiel für Substanz Nr. 1

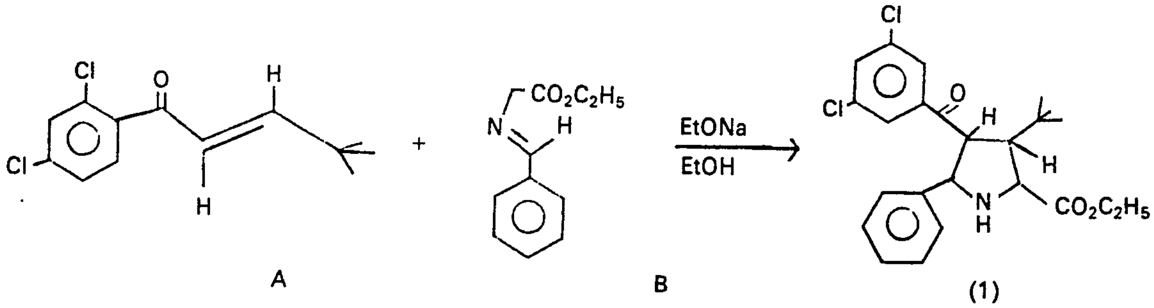


A B (1)

300 g (1,17 mol) A und 223 g (1,17 mol) B werden in 2 l trockenem Ethanol mit 8 g Natriumethylat unter Rühren 24 h bei Raumtemperatur und 48 h am Rückfluss gehalten. Der Umsatz wird mittels Dünnschichtchromatographie kontrolliert (Kieselgel, Methylenchlorid mit 5% Methanol als Laufmittel). Man kühlt auf 15 bis 20 °C ab und saugt kristallines (1) ab. Nach Waschen mit wenig kaltem Ethanol und Trocknen i.V. bei 50 °C erhält man 317 g (1) (Fp: 108–112 °C). Weitere 40 g (1) kristallieren aus der eingeengten Mutterlauge (Gesamtausbeute: 357 g, entsprechend (68% der rechnerisch möglichen Menge).

Herstellungsbeispiel für Substanz Nr. 3

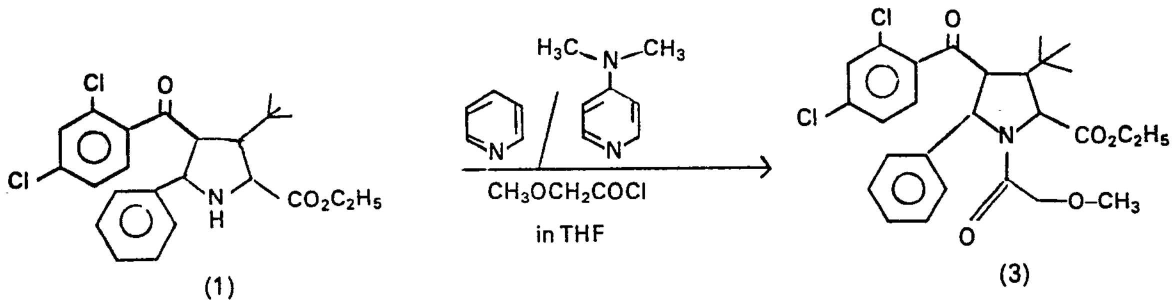


(1) (3)

Zu 20 g (0,045 mol) der wie vorstehend erhaltenen Verbindung (1), 3,62 ml (0,045 mol) Pyridin mit 10% 4-Dimethylaminopyridin in 200 ml trockenem THF werden 4,5 ml (0,049 ml) Methoxyessigsäurechlorid getropft. Nach 3 h bei Raumtemperatur wird eingedampft, in Methylenchlorid aufgenommen, je zweimal mit 1 n HCl, 5% wässrigem $NaHCO_3$ und Wasser gewaschen. Man trocknet mit $Na_2SO_4$ und dampft ein. Man erhält 21 g (90%) der Zielverbindung (3) als Öl, das beim Stehen kristallisiert. Fp: 136–140 °C.

Herstellungsbeispiel für Substanz Nr. 8

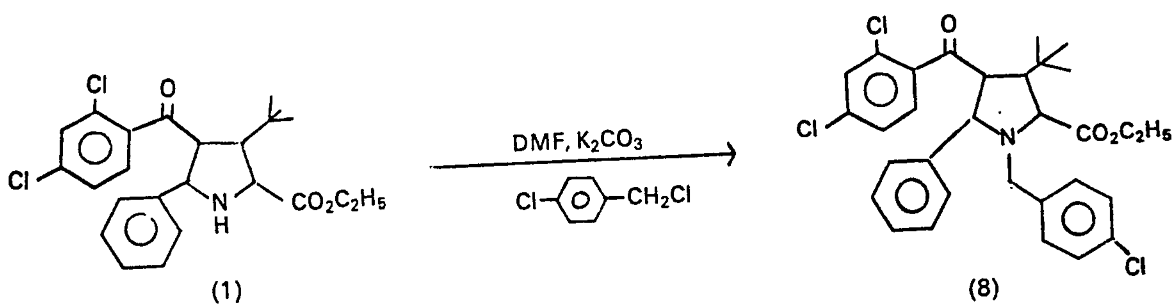


(1) (8)

20 g (0,045 mol) der Verbindung (1), 31 g $K_2CO_3$ und 9,2 ml (0,072 mol) 4-Chlorbenzylchlorid werden in 200 ml DMF 8 h bei 90 °C gerührt. Danach wird filtriert, eingedampft, in Methylenchlorid aufgenommen und mit Wasser gewaschen. Man trocknet mit $Na_2SO_4$ und dampft ein. Man reibt mit Pentan an und saugt ab: 19 g (74%) der Zielverbindung (8) (Fp: 176 bis 178 °C).

Herstellungsbeispiel für Substanz Nr. 11 (HCl-Salz)

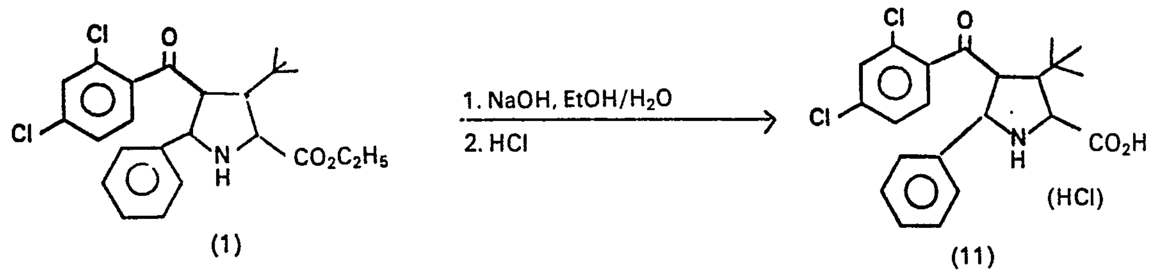


(1) (11)

15 g (0,033 mol) der Verbindung (1) werden in 100 ml 50 vol.-%igem wässrigen Ethanol mit 8,25 ml 4 n NaOH (0,033 mol) auf 70 bis 80 °C gehalten, bis im Dünnschichtchromatographen (Kieselgel, Methylenchlorid mit 5% Methanol als Laufmittel) kein Ester mehr zu erkennen ist. Man stellt mit konzentrierter Salzsäure auf pH 1, dampft ein, nimmt in Ethanol auf und filtriert ab. Beim Eindampfen der EtOH-Lösung kristallisiert die Zielverbindung aus. Nach Anpasten mit Pentan und Absaugen erhält man 7,1 g (47%) der Verbindung 11 der Tabelle (Fp: 142–146 °C).

Herstellungsbeispiel für Substanz Nr. 63

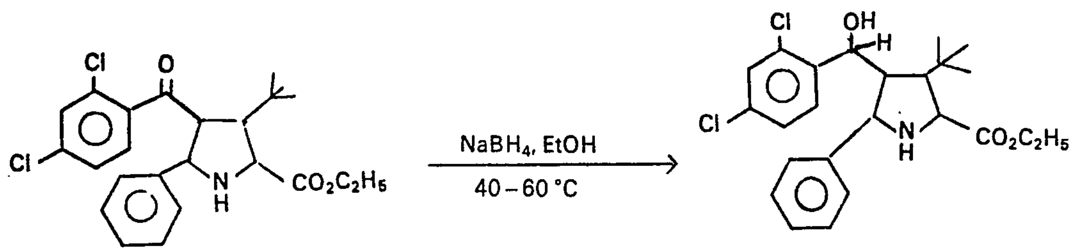


(1) (63)

98 g (0,219 mol) der Ausgangsverbindung (1) und 5 g (0,131 mol $NaBH_4$ werden in 1 l trockenem Ethanol bei 40 bis 60 °C gerührt bis im Dünnschichtchromatographen die Ausgangsverbindung verschwunden ist. Man filtriert, dampft ein, nimmt in $CH_2Cl_2$ auf, wäscht 3mal mit Wasser, trocknet mit $Na_2SO_4$ und dampft ein. Verreiben mit Ether und anschliessendes Absaugen liefert 48,3 g (49%) der Verbindung 63 (Fp: 117–124 °C).

Tabelle

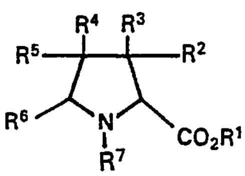

bzw. deren HCl-Salz

a) $R^2$=H, $R^4$=H, $R^6$=$C_6H_5$–, $R^3$=tert.-Butyl, $R^5$=CO–(2,4–$Cl_2$–$C_6H_3$)

| Nr. | $R^1$ | $R^7$ | Fp. [°C] |
|---|---|---|---|
| 1 | $-CH_2CH_3$ | –H | 108–112 |
| 2 | $-CH_2CH_3$ | $-COCH_2Cl$ | 118–122 |
| 3 | $-CH_2CH_3$ | $-COCH_2OCH_3$ | 136–140 |
| 4 | $-CH_2CH_3$ | –CO–($C_6H_5$) | 149–152 |
| 5 | $-CH_2CH_3$ | –CO–(3,5–$Cl_2$–$C_3H_6$) | 170–173 |
| 6 | $-CH_2CH_3$ | $-CH_2-CH=CH_2$ | wachs-artig |
| 7 | $-CH_2CH_3$ | $-CH_2-C_6H_5$ | 140–144 |
| 8 | $-CH_2CH_3$ | $-CH_2$–(4–Cl–$C_6H_4$) | 176–178 |
| 9 | $-CH_2CH_3$ | $-CH_2$–(4–F–$C_6H_4$) | 152–154 |
| 10 | $-CH_2H_3$ | $-CO_2C_2H_5$ | Öl |
| 11 | H | –H (HCl) | 142–146 |
| 12 | H | $-CH_2OCH_3$ | wachs-artig |
| 13 | H | $-CO-C_6H_5$ | 180–190 |
| 14 | H | –CO–(3,5–$Cl_2$–$C_6H_3$) | 187–191 |
| 15 | H | $-CH_2$–(4–Cl–$C_6H_4$) | 95–110 |

b) $R^2$=$R^4$=H, $R^6$=$C_6H_5$–, $R^3$=tert.-Butyl, $R^5$=–CO–(2–$OCH_3$–$C_6H_4$)

| Nr. | $R^1$ | $R^7$ | Fp. [°C] |
|---|---|---|---|
| 16 | $-CH_2CH_3$ | –H | 87– 88 |
| 17 | $-CH_2CH_3$ | $-COCH_2Cl$ | 94– 98 |
| 18 | $-CH_2CH_3$ | $-COCH_2OCH_3$ | 116–123 |
| 19 | $-CH_2CH_3$ | $-CO_2C_2H_5$ | Öl |
| 20 | –H | –H | 179–182 |
| 21 | –H | $-COCH_2OCH_3$ | 94– 96 |
| 22 | –H | $-CH_2-CH=CH_2$ | 62– 64 |
| 23 | $-CH_2CH_3$ | $-CH_2-CH=CH_2$ | 89– 94 |

c) $R^2$=$R^4$=H, $R^6$=$C_6H_5$, $R^3$=tert.-Butyl, $R^5$=–CO(4–$OC_3H_7$–$C_6H_4$)

| Nr. | $R^1$ | $R^7$ | Fp. [ °C] |
|---|---|---|---|
| 24 | $-CH_2CH_3$ | $-H$ | 85– 89 |
| 25 | $-CH_2CH_3$ | $-COCH_2OCH_3$ | 125–129 |
| 26 | $-CH_2CH_3$ | $-COCH_2Cl$ | 54– 60 |

d) $R^2=R^4=H$, $R^6=C_6H_5-$, $R^3$=tert.-Butyl, $R^5=-CO-(4-OC_4H_9-C_6H_4)$

| Nr. | $R^1$ | $R^7$ | Fp. [ °C] |
|---|---|---|---|
| 27 | $-CH_2CH_3$ | $-H$ | 88– 91 |
| 28 | $-CH_2CH_3$ | $-COCH_2Cl$ | 125–128 |
| 29 | $-CH_2CH_3$ | $-COCH_2OCH_3$ | 122–125 |
| 30 | $-CH_2CH_3$ | $-CH_2CH=CH_2$ | 116–118 |
| 31 | $-CH_2CH_3$ | $-CH_2C_6H_5$ | 142–144 |
| 32 | $-CH_2CH_3$ | $-CH_2-(4-Cl-C_6H_4)$ | 180–183 |

e) $R^2=R^4=H$, $R^6=C_6H_5-$, $R^3$=tert.-Butyl, $R^5=-CO-(2-Cl-C_6H_4)$

| Nr. | $R^1$ | $R^7$ | Fp. [ °C] |
|---|---|---|---|
| 33 | $-C_2H_5$ | H | 75– 81 |
| 34 | $-C_2H_5$ | $-CH_2-(4-Cl-C_6H_4)$ | 155–158 |
| 35 | $-C_2H_5$ | $-CH_2CH=CH_2$ | wachs- |
| 36 | $-C_2H_5$ | $-CO-(3,5-Cl_2-C_6H_3)$ | artig |
| 37 | $-C_2H_5$ | $-COCH_2Cl$ | 140–144 |
| 38 | $-C_2H_5$ | $-COOC_2H_5$ | Öl |
| 39 | $-C_2H_5$ | $-COCH_2OCH_3$ | Öl |
| 40 | $-H$ | $-COCH_2OCH_3$ | Öl |
| 41 | $-H$ | $-H$ (HCl) | 88– 93 |
| 42 | $-H$ | $-CO-(3,5-Cl_2-C_6H_3)$ | 231–233 |
| | | | 146–154 |

f) $R^2=R^4=H$, $R^6=C_6H_5-$, $R^3$=tert.-Butyl, $R^5=-CO-(2,5-Cl_2-C_6H_3)$

| Nr. | $R^1$ | $R^7$ | Fp. [ °C] |
|---|---|---|---|
| 43 | $-C_2H_5$ | H | 105–109 |
| 44 | $-C_2H_5$ | $-COC_6H_5$ | 162–166 |
| 45 | $-C_2H_5$ | $-CO-CH_2OCH_3$ | Öl |
| 46 | $-C_2H_5$ | $-CO-(3,5-Cl_2-C_6H_3)$ | 184–186 |
| 47 | $-C_2H_5$ | $-CO_2C_2H_5$ | Öl |
| 48 | $-C_2H_5$ | $-COCH_2Cl$ | Öl |
| 49 | $-C_2H_5$ | $-CH_2-CH=CH_2$ | Öl |
| 50 | $-C_2H_5$ | $-CH_2-C_6H_5$ | 117–121 |
| 51 | $-C_2H_5$ | $-CH_2-(4-F-C_6H_4)$ | 130–136 |
| 52 | $-C_2H_5$ | $-CH_2-(4-Cl-C_6H_4)$ | 150 |
| 53 | H | $-CO-C_6H_5$ | 191–197 |
| 54 | H | $-COCH_2OCH_3$ | 85– 90 |
| 55 | H | $-CO-(3,5-Cl_2-C_6H_3)$ | 180–117 |
| 56 | H | $-COCH_2Cl$ | 109–112 |
| 57 | H | $-CH_2-C_6H_5$ | 135–140 |
| 58 | H | $-CH_2-(4-F-C_6H_4)-H$ (HCl) | 170–200 (Zers.) |
| 59 | H | $-CH_2-(4-Cl-C_6H_4)-H$ (HCl) | 131–137 |
| 60 | H | $-H$ (HCl) | 110 (Zers.) |
| 61 | H | $-CH_2CH=CH_2-H$ (HCl) | 200 |

g) $R^2=R^4=H$, $R^6=C_6H_5-$, $R^3$=tert.-Butyl, $R^5=-CHOH-R^8$, $R^8=2,4-Cl_2-C_6H_3$,

| Nr. | $R^1$ | $R^7$ | Fp. [ °C] |
|---|---|---|---|
| 62 | $-CH(CH_3)_2$ | H | 205–217 |
| 63 | $-C_2H_5$ | H | 117–124 |
| 64 | $-C_2H_5$ | $-COCH_2Cl$ | 89– 98 |
| 65 | $-C_2H_5$ | $-CH_2OCH_3$ | 142–150 |
| 66 | $-C_2H_5$ | $-CO_2C_2H_5$ | Öl |
| 67 | $-C_2H_5$ | $-CH_2-CH=CH_2$ | 131–136 |
| 68 | $-C_2H_5$ | $-CH_2-(4-Cl-C_6H_4)$ | 100–110 |
| 69 | H | $-H$ (HCl) | 259–265 |
| 70 | H | $-COCH_2Cl$ | 165–170 |
| 71 | H | $-COCH_2OCH_3$ | 137–146 |
| 72 | H | $-CH_2-(4-Cl-C_6H_4)$ | 237–239 |

h) $R^2=H$, $R^6=-C_6H_5$, $R^3=-(4-NO_2-C_6H_4)$, $R^5=-CO-C_6H_5$

| Nr. | $R^1$ | $R^4$ | $R^7$ | Fp. [ °C] |
|---|---|---|---|---|
| 73 | $-C_2H_5$ | $-CH_3$ | H | 153–155 |
| 74 | $-H$ | $-CH_3$ | H | |
| 75 | $-C_2H_5$ | $-CH_3$ | $-CH_2-C_6H_5$ | |
| 76 | $-C_2H_5$ | $-CH_3$ | $-CH_2-(4-Cl-C_6H_4)$ | |
| 77 | $-C_2H_5$ | $-CH_3$ | $-CO-C_6H_5$ | |
| 78 | $-C_2H_5$ | $-CH_3$ | $-CO-(3,5-Cl_2-C_6H_3$ | |
| 79 | $-C_2H_5$ | $-CH_3$ | $-COCH_2Cl$ | |
| 80 | $-C_2H_5$ | $-CH_3$ | $-COCH_2OCH_3$ | |
| 81 | H | $-CH_3$ | $-CH_2-C_6H_5$ | |
| 82 | H | $-CH_3$ | $-CH_2-(4-Cl-C_6H_4)$ | |
| 83 | H | $-CH_3$ | $-CO-C_6H_5$ | |

| Nr. | $R^1$ | $R^4$ | $R^7$ | Fp. [°C] |
|---|---|---|---|---|
| 84 | H | $-CH_3$ | $-CO-(4-Cl-C_6H_4)$ | |
| 85 | H | $-CH_3$ | $-COCH_2Cl$ | |
| 86 | H | $-CH_3$ | $-COCH_2OCH_3$ | |

i) $R^2$=H, $R^5$=CN, $R^4$=–1–(1,2,4-Triazolyl)

| Nr. | $R^1$ | $R^3$ | $R^6$ | $R^7$ | Fp. [°C] |
|---|---|---|---|---|---|
| 87 | $C_2H_5$ | $-(2,4-Cl_2-C_6H_3)$ | $-C_6H_5$ | H | 185 |
| 88 | $C_2H_5$ | $-(4-Cl-C_6H_4)$ | $-C_6H_5$ | H | |
| 89 | $C_2H_5$ | $-C_6H_5$ | $-C_6H_5$ | H | |
| 90 | $C_2H_5$ | $-(2,4-Cl_2-C_6H_3)$ | $-(2,4-Cl_2-C_6H_3)$ | H | |
| 91 | $C_2H_5$ | $-C_6H_5$ | $-(2,4-Cl_2-C_6H_3)$ | H | |
| 92 | $C_2H_5$ | $-(2,4-Cl_2-C_6H_3)$ | $-C_6H_5$ | $-COCH_2Cl$ | |
| 93 | $C_2H_5$ | $-(2,4-Cl_2-C_6H_3)$ | $-C_6H_5$ | $-COCH_2OCH_3$ | |
| 94 | $C_2H_5$ | $-(2,4-Cl_2-C_6H_3)$ | $-C_6H_5$ | $-CO-C_6H_5$ | |
| 95 | $C_2H_5$ | $-(2,4-Cl_2-C_6H_3)$ | $-C_6H_5$ | $-CO(4-ClC_6H_4)$ | |
| 96 | $C_2H_5$ | $-(2,4-Cl_2-C_6H_3)$ | $-C_6H_5$ | $-CO-(3,5-Cl_2-C_6H_3)$ | |
| 97 | H | $-(2,4-Cl_2-C_6H_3)$ | $-C_6H_5$ | $-COCH_2Cl$ | |
| 98 | H | $-(2,4-Cl_2-C_6H_3)$ | $-C_6H_5$ | $-COCH_2OCH_3$ | |

## Patentansprüche

1. Pyrrolidincarbonsäurederivate der Formel I

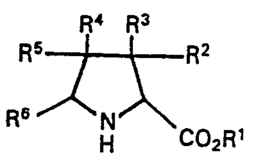

I,

in welcher

$R^1$, $R^2$, $R^3$ unabhängig voneinander für Wasserstoff, nieder-Alkyl mit bis zu 5 Kohlenstoffatomen, nieder-Halogenalkyl mit bis zu 3 Kohlenstoffatomen und bis zu 5 Halogenatomen, Phenylalkyl mit bis zu 4 Kohlenstoffatomen im Alkylteil, Halogenphenylalkyl mit bis zu 3 Halogenatomen im Phenylteil und bis zu 4 Kohlenstoffatomen im Alkylteil stehen,

$R^2$, $R^3$ darüber hinaus unabhängig voneinander für substituiertes Phenyl, wobei als Substituenten bis zu 3 Halogenatome, eine Nitro-, eine Cyano- oder eine Alkoxygruppe mit bis zu 4 Kohlenstoffatomen gemeint sind, stehen, und darüber hinaus $R^3$ auch für unsubstituiertes Phenyl stehen kann,

$R^4$ für Wasserstoff, n- oder i-Alkyl mit bis zu 5 Kohlenstoffatomen, Phenylalkyl mit bis zu 4 Kohlenstoffatomen im Alkylteil, Halogenphenylalkyl mit bis zu 3 Halogenatomen im Phenylteil und bis zu 4 Kohlenstoffatomen im Alkylteil, Phenyl oder substituiertes Phenyl, wobei als Substituenten bis zu 3 Halogenatome, eine Nitro- eine Cyano- oder eine Alkoxygruppe mit bis zu 4 Kohlenstoffatomen gemeint sind, einen fünf- bis sechsatomigen aromatischen Heterocyclus mit bis zu 3 Stickstoffen als Heteroatomen, eine Cyanogruppe, eine Alkylcarbonylgruppe mit bis zu 5 Kohlenstoffatomen, eine Halogenalkylcarbonylgruppe mit bis zu 3 Kohlenstoffatomen und bis zu 5 Halogenatomen oder eine Alkoxycarbonylgruppe mit bis zu 5 Kohlenstoffatomen steht,

$R^5$ für Alkylcarbonyl mit bis zu 5 Kohlenstoffatomen, Halogenalkylcarbonyl mit bis zu 3 Kohlenstoffatomen und bis zu 5 Halogenatomen, Alkoxycarbonyl mit bis zu 5 Kohlenstoffatomen, gegebenenfalls substituiertes Phenylcarbonyl, wobei als Substituenten bis zu 3 Halogenatome, eine Alkoxygruppe mit bis zu 4 Kohlenstoffatomen, eine Alkylthiogruppe mit bis zu 4 Kohlenstoffatomen, eine Cyano- oder eine Nitrogruppe gemeint sind, oder für eine Cyano- oder eine Nitrruppe steht,

$R^6$ für gegebenenfalls substituiertes Phenyl, wobei als Substituenten bis zu 3 Halogenatome, eine Alkoxygruppe mit bis zu 4 Kohlenstoffatomen, eine Alkylthiogruppe mit bis zu 4 Kohlenstoffatomen, eine Alkylgruppe mit bis zu 5 Kohlenstoffatomen, eine Cyano- oder eine Nitrogruppe gemeint sind, oder tert. Alkyl mit bis zu 5 Kohlenstoffatomen steht.

2. Pyrrolidine der Formel Ia

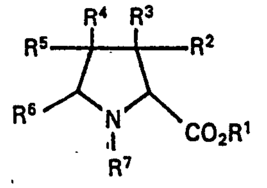

Ia,

in welcher $R^1$ bis $R^6$ die im Anspruch 1 angegebene Bedeutung haben und $R^7$ nieder-Alkyl mit bis zu 5 Kohlenstoffatomen, nieder-Alkenyl mit bis 5 Kohlenstoffatomen, das mit bis zu 3 Halogenatomen substituiert ist, nieder-Alkinyl mit bis zu 5-Kohlenstoffatomen, gegebenenfalls substituiertes Phenylalkyl mit bis zu 3 Kohlenstoffatomen im Alkylteil und bis zu 3 Halogenatomen, einer Cyano- oder einer Nitrogruppe als Substituenten im Phenylteil, ggf. subst. Alkylcarbonyl mit bis zu 5 Kohlenstoffatomen und bis 3 Halogenatomen oder einer Alkoxygruppe ($C_1$–$C_3$) als Substituenten, Alkoxycarbonyl mit bis zu 5 Kohlenstoffatomen, gegebenenfalls substituiertes Phenylcarbonyl mit bis zu 3 Halogenatomen als Substituenten, Phenylalkylcarbonyl mit bis zu 3 Kohlenstoffen im Alkylteil oder Benzyloxycarbonyl bedeutet.

3. Pyrrolidine der Formel Ib

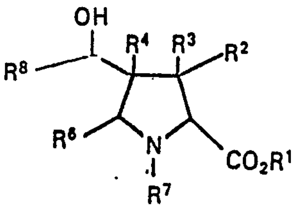

Ib,

in welcher $R^1$, $R^2$, $R^3$, $R^4$, $R^6$, $R^7$ die in Anspruch 1 bzw. 2 angegebene Bedeutung haben, mit der Massgabe, dass $R^7$ auch H sein kann und mit der Massgabe, dass $R^4$ nur für n- oder i-Alkyl, ggf. subst. Phenylalkyl, ggf. subst. Phenyl, einen fünf- bis sechsgliedrigen aromatischen Heterocyclus mit bis zu 3 Stickstoffatomen oder eine Cyanogruppe steht, und in welcher $R^8$ für Alkyl oder Halogenalkyl mit bis zu 4 Kohlenstoffatomen und bis zu 3 Halogenatomen oder gegebenenfalls substituiertes Phenyl steht, wobei als Substituenten bis zu 3 Halogenatome, eine Alkoxygruppe mit bis zu 4 Kohlenstoffatomen, eine Alkylthiogruppe mit bis zu 4 Kohlenstoffatomen, eine Cyano- oder eine Nitrogruppe gemeint sind.

4. Verfahren zur Herstellung der Verbindungen der Formel I nach Anspruch 1, dadurch gekennzeichnet, dass man an entsprechende Alkene der Formel IV

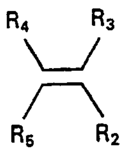

(IV)

entsprechende Schiff-Basen von Glycinestern der Formel V

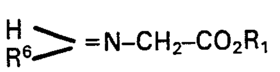

(V)

in Gegenwart einer Base und ggf. in einem Verdünnungsmittel, anlagert.

5. Verfahren nach Anspruch 4, dadurch gekennzeichnet, dass man als Verdünnungsmittel Ethanol und als Base Natriumethylat verwendet und die Umsetzung/Anlagerung bei einer Temperatur von oberhalb 60 °C vornimmt.

6. Verfahren zur Herstellung der Verbindungen der Formel Ia nach Anspruch 2, dadurch gekennzeichnet, dass man Verbindungen der Formel I mit einem entsprechenden Alkylierungs- oder Acylierungsmittel $R^7X$ umsetzt, wobei X im Falle eines Alkylierungsmittels für eine Fluchtgruppe wie Cl, Br, I, Tosylat, Mesylat oder Sulfat und im Falle eines Acylierungsmittels für eine aktivierte Carbonsäuregruppe CO–Y steht, in welcher Y Cl, Br, Imidazolyl, $CF_3CO_2$ oder $C_1$– bis $C_4$-Monoalkylcarbonat bedeutet und ggf. eines Verdünnungsmittels durchgeführt wird. Verdünnungsmittel sind dabei niedere Alkohole ($C_1$ bis $C_4$), DMSO, DMF, N-Methylpyrrolidon oder ähnliche organische Lösungsmittel.

7. Verfahren zur Herstellung der Verbindungen der Formel Ib nach Anspruch 3, dadurch gekennzeichnet, dass man entsprechende Verbindungen der Formel (I) bzw. (Ia) reduziert – mit der Massgabe, dass $R^4$ nur für n- oder i-Alkyl, ggf. subst. Phenylalkyl, ggf. subst. Phenyl, einen fünf- bis sechsgliedrigen aromatischen Heterocyclus mit bis zu 3 Stickstoffatomen oder eine Cyanogruppe steht, und mit der Massgabe, dass $R^5$ nur für ggf. subst. Alkylcarbonyl oder ggf. subst. Phenylcarbonyl steht –, indem man mit einem komplexen Metall-Hydrid umsetzt.

**Revendications**

1. Dérivés d'acide pyrolidine-carboxylique de formule 1 dans laquelle

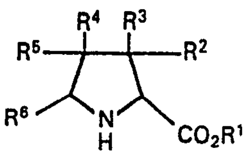

I,

$R^1$, $R^2$, $R^3$ sont mis, indépendamment l'un de l'autre pour hydrogène, alkyle inférieur ayant jusqu'à 5 atomes de carbone, halogéno-alkyle inférieur ayant jusqu'à 3 atomes de carbone et jusqu'à 5 atomes d'halogène, phenylalkyle ayant jusqu'à atomes de carbone dans la partie alkyle, halogénophénylalkyle ayant jusqu'à 3 atomes d'halogène dans la partie phényle et jusqu'à 4 atomes de carbone dans la partie alkyle.

$R^2$, $R^3$, en outre, représentent, indépendamment l'un de l'autre, phényle substitué, en prenant comme substituants jusqu'à 3 atomes d'halogène un groupe nitro, un groupe cyano ou un groupe alcoxy ayant jusqu'à 4 atomes de carbone et, en outre, $R^3$ étant mis aussi comme substituant,

$R^4$ est mis pour hydrogène n-alkyle ou i-akyle ayant jusqu'à 5 atomes de carbone, phénylalkyle ayant jusqu'à 4 atomes de carbone dans la partie alkyle halogéno-phénylalkyle ayant jusqu'à 3 atomes d'halogène dans la partie phényle et jusqu'à 4 atomes de carbone dans la partie alkyle, phényle ou phényle substitué, en entendant comme substituants, jusqu'à 3 atomes d'halogène un groupe nitro, un groupe cyano ou un groupe alcoxy ayant jusqu'à 4 atomes de carbone, un hétérocycle aromatique d'atomicité cinq ou six, avec jusqu'à 3 azotes comme hétéroatomes, un groupe cyano, un groupe alkylcarbonyle ayant jusqu'à 5 atomes de carbone, un groupe halogénoalkyl-carbonyle

ayant jusqu'à 3 atomes de carbone et jusqu'à 5 atomes d'halogène ou un groupe alkyloxycarbonyle ayant jusqu'à 5 atomes de carbone.

$R^5$ représente alkylcarbonyle ayant jusqu'à 5 atomes de carbone, halogénalkylcarbonyle ayant jusqu'à 3 atomes de carbone et jusqu'à 5 atomes d'halogène alcoxycarbonyle ayant jusqu'à 5 atomes de carbone phénylcarbonyle éventuellement substitué, en entendant comme substituants jusqu'à 3 atomes d'halogène, un groupe alcoxy ayant jusqu'à 4 atomes de carbone, un groupe alkylthio ayant jusqu'à 4 atomes de carbone, un groupe cyano ou un groupe nitro, ou représente un groupe cyano ou groupe nitro,

$R^6$ est mis pour phényle éventuellement substitué en entendant, comme substituants, jusqu'à 3 atomes d'halogène, un groupe alcoxy ayant jusqu'à 4 atomes de carbone, un groupe alkylthio ayant jusqu'à 4 atomes de carbone, un groupe alkyle ayant jusqu'à 5 atomes de carbone, un groupe cyano ou un groupe nitro, ou représente alkyle tert. ayant jusqu'à 5 atomes de carbone.

2. Pyrolidine de formule 1a

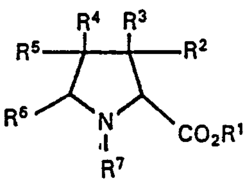

1a

dans laquelle $R^1$ à $R^6$ ont les significations données dans la revendication 1 et $R^7$ signifie alkyle inférieur ayant jusqu'à 5 atomes de carbone, alcényle inférieur ayant jusqu'à 5 atomes de carbone, qui est substitué par jusqu'à 3 atomes d'halogène, alcynyle-inférieur ayant jusqu'à 5 atomes de carbone, phénylalkyle, éventuellement substitué, ayant jusqu'à 3 atomes de carbone dans la partie alkyle et jusqu'à 3 atomes d'halogène, un groupe cyano ou un groupe nitro comme substituants dans la partie phényle alkylcarbonyle éventuellement substitué ayant jusqu'à 5 atomes de carbone et jusqu'à 3 atomes d'halogène ou un groupe alcoxy ($C_1$–$C_3$) comme substituants, alcoxycarbonyle ayant jusqu'à 5 atomes de carbone phénylcarbonyle, éventuellement substitué, ayant jusqu'à 3 atomes d'halogène comme substituants phénylalkylcarbonyle ayant jusqu'à 3 atomes de carbone dans la partie alkyle, ou benzyloxycarbonyle.

3. Pyrolidine de formule Ib

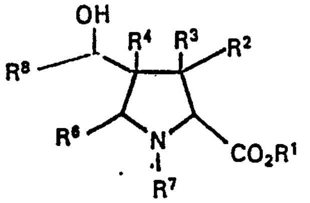

Ib

dans laquelle $R^1$, $R^2$, $R^3$, $R^4$, $R^6$, $R^7$ ont les significations données dans la revendication 1 ou 2, avec la condition que $R^7$ peut être aussi H et avec la condition que $R^4$ ne représente que n- ou i-alkyle, phénylalkyle éventuellement substitué, phényle éventuellement substitué, un hétérocycle à cinq ou six chaînons, ayant jusqu'à 3 atomes d'azote ou un groupe cyano, et dans laquelle $R^8$ est mis pour alkyle ou halogénalkyle ayant jusqu'à 4 atomes de carbone et jusqu'à 3 atomes d'halogène ou phényle éventuellement substitué, en entendant comme substituants jusqu'à 3 atomes d'halogène, un groupe alcoxy ayant jusqu'à 4 atomes de carbone, un groupe alkylthio ayant jusqu'à 4 atomes de carbone, un groupe cyano ou un groupe nitro.

4. Procédé de préparation des composés de formule 1 selon la revendication 1, caractérisé par le fait que l'on fixe par addition, sur des alcènes correspondants de formule IV

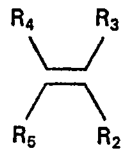

(IV)

des bases de Schiff correspondants de glycine-esters de formule V

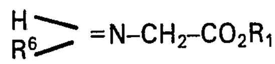

(V)

en présence d'une base et éventuellement un diluant.

5. Procédé selon la revendication 4, caractérisé par le fait que l'on utilise comme diluant, de l'éthanol et comme base, du méthylate de sodium et la réaction/addition s'effectue à une température supérieure à 60 °C.

6. Procédé de préparation de composés de formule la selon la revendication 2, caractérisé par le fait que l'on fait réagir des composés de formule I avec un agent alkylation ou d'acylation $R^7X$ correspondant, X étant mis, dans le cas d'un agent alkylation, pour un groupe de fuite tel que Cl, Br, I, tosylate, mésylate ou sulfate et, dans le cas d'un agent d'acylation, pour un groupe acide carboxylique active CO–Y, dans lequel Y représente Cl, Br, imidazolyle, $CF_3CO_2$ ou monoalkylcarbonate en $C_1$ à $C_4$ et éventuellement en présence d'un diluant, des diluants sont ici des alcools inférieurs ($C_1$ à $C_4$), DMSO, DMF, N-méthylpyrolidine ou des solvants organiques analogues.

7. Procédé de préparation des composés de formule Ib selon la revendication 3, caractérisé par le fait que l'on réduit des composés correspondants de formule (I) ou (Ia), sous réserve que $R^4$ ne soit mis que pour n- ou i-alkyle phénylalkyle éventuellement substitué, phényle éventuellement substitué, un hétérocycle aromatique à cinq ou six chaînons, ayant jusqu'à 3 atomes d'azote ou un groupe cyano et sous réserve que $R^5$ ne soit mis que pour alkylcarbonyle éventuellement substitué, ou phénylcarbonyle éventuellement substitué en faisant réagir avec un hydrure de métal complexe.

**Claims**

1. A pyrrolidinecarboxylic acid derivative of the formula I

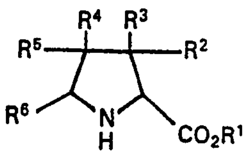

I,

where

$R^1$, $R^2$ and $R^3$ independently of one another are each hydrogen, lower alkyl of not more than 5 carbon atoms, lower haloalkyl of not more than 3 carbon atoms and not more than 5 halogen atoms, phenylalkyl where alkyl is of not more than 4 carbon atoms, halophenylalkyl where the phenyl moiety has not more than 3 halogen atoms and alkyl is of not more than 4 carbon atoms,

$R^2$ and $R^3$ moreover independently of one another are each substituted phenyl, substituents being up to 3 halogen atoms, a nitro group, a cyano group or an alkoxy group of not more than 4 carbon atoms, and furthermore $R^3$ may also be unsubstituted phenyl,

$R^4$ is hydrogen, n- or isoalkyl of not more than 5 carbon atoms, phenylalkyl where alkyl is of not more than 4 carbon atoms, halophenylalkyl where the phenyl moiety has not more than 3 halogen atoms and alkyl is of not more than 3 carbon atoms, phenyl or substituted phenyl, substituents being up to 3 halogen atoms, a nitro group, a cyano group or an alkoxy group of not more than 4 carbon atoms, a five-membered or six-membered aromatic heterocycle having not more than 3 nitrogens as heteroatoms, cyano, alkylcarbonyl of not more than 5 carbon atoms, haloalkylcarbonyl of not more than 3 carbon atoms and not more than 5 halogen atoms or alkoxycarbonyl of not more than 5 carbon atoms,

$R^5$ is alkylcarbonyl of not more than 5 carbon atoms, haloalkylcarbonyl of not more than 3 carbon atoms and not more than 5 halogen atoms, alkoxycarbonyl of not more than 5 carbon atoms, unsubstituted or substituted phenylcarbonyl, substituents being up to 3 halogen atoms, alkoxy of not more than 4 carbon atoms, alkylthio of not more than 4 carbon atoms, cyano or nitro, or $R^5$ is cyano or nitro, and

$R^6$ is unsubstituted or substituted phenyl, substituents being up to 3 halogen atoms, alkoxy of not more than 4 carbon atoms, alkylthio of not more than 4 carbon atoms, alkyl of not more than 5 carbon atoms, cyano or nitro, or $R^6$ is tert-alkyl of not more than 5 carbon atoms.

2. A pyrrolidine of the formula Ia

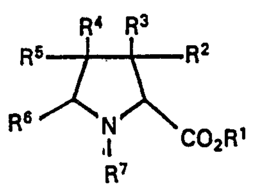

Ia,

where $R^1$ to $R^6$ have the meanings stated in claim 1 and $R^7$ is a lower alkyl of not more than 5 carbon atoms, lower alkenyl of not more than 5 carbon atoms which is substituted by not more than 3 halogen atoms, lower alkynyl of not more than 5 carbon atoms, unsubstituted or substituted phenylalkyl which has not more than 3 carbon atoms in the alkyl moiety and not more than 3 halogen atoms, a cyano group or a nitro group as substituents in the phenyl moiety, unsubstituted or substituted alkylcarbonyl which is of not more than 5 carbon atoms and has up to 3 halogen atoms or a $C_1$–$C_3$-alkoxy group as substituents, alkoxycarbonyl of not more than 5 carbon atoms, unsubstituted or substituted phenyl carbonyl having not more than 3 halogen atoms as substituents, phenylalkylcarbonyl where alkyl is of not more than 3 carbon atoms or benzyloxycarbonyl.

3. A pyrrolidine of the formula Ib

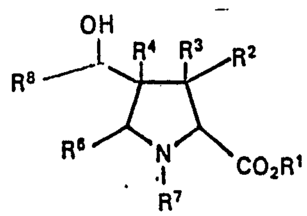

Ib,

where $R^1$, $R^2$, $R^3$, $R^4$, $R^6$ and $R^7$ have the meanings stated in claim 1 or 2, with the proviso that $R^7$ may furthermore be H and with the proviso that $R^4$ is only n- or isoalkyl, unsubstituted or substituted phenylalkyl, unsubstituted or substituted phenyl, a five-membered or six-membered aromatic heterocycle having not more than 3 nitrogen atoms or a cyano group, and in which $R^8$ is alkyl or haloalkyl of not more than 4 carbon atoms and not more than 3 halogen atoms, or unsubstituted or substituted phenyl, substituents being up to 3 halogen atoms, alkoxy of not more than 4 carbon atoms, alkylthio of not more than 4 carbon atoms, cyano or nitro.

4. A process for the preparation of a compound of the formula I as claimed in claim 1, wherein an appropriate Schiff's base of a glycine ester of the formula V

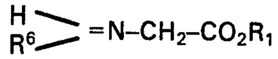

(V)

is subjected to an addition reaction with an appropriate alkene of the formula IV

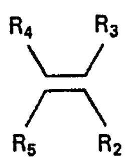

(IV)

in the presence of a base and in the presence or absence of a diluent.

5. A process as claimed in claim 4, wherein ethanol is used as a diluent and sodium ethylate as a base, and the reaction/addition reaction is carried out at above 60 °C.

6. A process for the preparation of a compound

of the formula Ia as claimed in claim 2, wherein a compound of the formula I is reacted with an appropriate alkylating or acylating agent $R^7X$, where X is a leaving group, such as Cl, Br, I, tosylate, mesylate or sulfate, in the case of an alkylating agent and is an activated carboxylic acid group CO–Y, where Y is Cl, Br, imidazolyl, $CF_3CO_2$ or $C_1$–$C_4$-monoalkylcarbonate, in the case of an acylating agent, and is carried out in the presence or absence of a diluent, diluents being lower alcohols ($C_1$–$C_4$), DMSO, DMF, N-methylpyrrolidone or similar organic solvents.

7. A process for the preparation of a compound of the formula Ib as claimed in claim 3, wherein an appropriate compound of the formula (I) or (Ia) is reduced, with the proviso that $R^4$ is only n- or isoalkyl, unsubstituted or substituted phenylalkyl, unsubstituted or substituted phenyl, a five-membered or six-membered aromatic heterocycle having not more than 3 nitrogen atoms, or cyano, and with the proviso that $R^5$ is only unsubstituted or substituted alkylcarbonyl or unsubstituted or substituted phenylcarbonyl, by reaction with a complex metal hydride.